Europäisches Patentamt

**European Patent Office**

**Office européen des brevets**

(19)

(11) Publication number: **0 128 665**
A2

(12)

# EUROPEAN PATENT APPLICATION

(21) Application number: **84303153.5**

(22) Date of filing: **10.05.84**

(51) Int. Cl.³: **C 21 C 5/46**, C 21 C 5/30, G 01 N 27/56

(30) Priority: **14.05.83 GB 8313343**

(43) Date of publication of application: **19.12.84**
**Bulletin 84/51**

(84) Designated Contracting States: **AT BE CH DE FR GB IT LI LU NL SE**

(71) Applicant: **Hamblin, Michael Jonathan, 7 York Street, East Markham nr. Newark NG22 OQW (GB)**
Applicant: **Nixon, James Herbert, 66 Brookhouse Hill, Fulwood Sheffield, S10 3TB (GB)**
Applicant: **Bingham, Douglas Fields, 8 Moorbank Close, Sheffield, 10 (GB)**

(72) Inventor: **Hamblin, Michael Jonathan, 7 York Street, East Markham nr. Newark NG22 OQW (GB)**
Inventor: **Nixon, James Herbert, 66 Brookhouse Hill, Fulwood Sheffield, S10 3TB (GB)**
Inventor: **Bingham, Douglas Fields, 8 Moorbank Close, Sheffield, 10 (GB)**

(74) Representative: **Houghton, David et al, Hulse & Co. Cavendish Buildings West Street, Sheffield, S1 1ZZ (GB)**

(54) **Heat resistant means.**

(57) The invention relates to heat resistant means such as an immersion tube for temperature measurement or sampling of a bath of molten metal. Immersion tubes are known to be made from conventional refractory materials, but are expensive. To reduce costs, it is known to employ cardboard tubes, but this invariably results in splashing of molten metal on immersion in the bath, and immersion time is very limited before the tube is burned away, with the possible loss of the sampler or the pyrometer fitted in the tube. The object of the invention is to avoid these disadvantages, which objective is met by a heat resistant means having a surface formed from or coated with an ablative or decomposible material, for example, an admixture of inorganic spheroidal powder, granular refractory material and/or refractory fibrous material and an inorganic binder.

ACTORUM AG

## HEAT RESISTANT MEANS

This invention relates to heat resistant means and is particularly, but not exclusively, concerned with heat resistant means for insertion into a bath of liquid metal.

There are many instances where a device for effecting a particularly function must be introduced into a hostile atmosphere. Thus, for example, in steel making there is the frequent necessity for either the taking of a sample of liquid steel from a furnace or a ladle or the introduction into the furnace or the ladle of a pyrometer for sensing the temperature of the molten metal. To allow the particular device to effect its required function, there must be provided some form of protection against the hostile environment into which it is introduced. Again taking steel making as a example, it is known for sampling devices or for pyrometers to be located towards the end of a lance with the lance formed from a heat resistant material. When using refractory materials proper, such lances are expensive and as a means of reducing costs it is already known to provide such sampler or pyrometer towards the end of a cardboard tube of appropriate length. Whilst this reduces noticeably the cost of sampling or the

- 2 -                           0128665

cost of obtaining temperature, such constructions have two principle disadvantages, one being inevitable splashing of molten metal as the cardboard tube is immersed stemming from the inevitable water content of the cardboard and the virtual instantaneous emission of water vapour as the cardboard is rapidly elevated in temperature, and the other stems from the inherent combustible nature of cardboard and its essential limitation on the immersion time, and which is unavoidably extremely low before the cardboard tube is destroyed with the consequent loss of the sampler or the pyrometer.

The object of the present invention is to provide a heat resistant means in which the above inherent disadvantages of known devices are avoided.

According to the present invention a heat resistant means comprises a surface formed from or overlaid with an ablative or decomposible refracting material. Thus, by forming the surface from or overlaying the surface with such material, the effect of introduction of said means into a hostile high temperature environment is that the surface of the material ablates or decomposes at a temperature below that of the environment, and the thermal diffusivity of the composite of surface ablated or decomposed material and unablated or undecomposed remainder of the material, is sufficiently low that the mechanical strength of said means is maintained to a sufficient extent to avoid mechanical failure during the period of required exposure of said means in said environment.

In its application to the sampling or temperature sensing of liquid metal, said material can be formed as a tube with the sampler and/or pyrometer set towards the end of such tube. Alternatively an ablative material may be used as a coating on a cardboard support tube. By its very nature the material can be pre-heated to a substantial degree, and accordingly the problems of splashing inherent with cardboard tubes is avoided and at the same time such tube can be held submerged in molten metal for at least an equal period.

The ablative or decomposible refracting material can be any one or more of a material of low thermal conductivity or a dense material of high thermal capacity. Thus, the composition of the material may be an inorganic hollow spheroidal powder mixed with a proportion of granular

- 4 -

0128665

refractory powder, and optionally refractory fibrous material, bonded by an inorganic binder such as sodium or calcium silicate. A typical composition would be an inorganic hollow spheroidal powder in the amount 30% to 70% by weight, a granular refractory powder in the amount 0 to 30% by weight, a refractory fibrous material 0 to 10% by weight, and sodium silicate solution 5% to 50% by weight. Preferably the hollow spheroidal powder is 50% by weight, the granular refractory powder is 10% by weight, and the sodium silicate solution 40% by weight.

The inorganic hollow spheroidal powder may have an aluminia/silica base, the granular material may be silica sand, and the fibrous material may be glass fibre, or rock wool fibre. The material utilised in one of several relatively conventional techniques for forming shapes of refractory materials, to form the material into a tube, and when a coating to a cardboard support tube, applied by any relatively conventional technnique such as dip coating.

For greater effectiveness, it is preferred that the material forming or overlaying the surface is cured or fired prior to introduction into the hostile environment.

0128665

The result of the invention is a highly effective heat resistant means for insertion into a bath of liquid metal, in terms of both its costs of production and its performance.

CLAIMS

1. A heat resistant means comprising a surface formed from or overlaid with an ablative or decomposible refracting material.

2. A heat resistant means as in Claim 1, is formed as a tube from said ablative or decomposible material.

3. A heat resistant means as in Claim 1, is formed as a tube of cardboard coated with said ablative or decomposible material.

4. A heat resistant means as in Claim 3, wherein said coating is a dip coating.

5. A heat resistant means as in any of Claims 1 to 4, wherein said ablative or decomposible material is a material of low thermal conductivity or high thermal capacity.

6. A heat resistant means as in Claim 5, wherein the said material is a hollow spheroidal powder mixed with a granular refractory powder, and/or refractory fibrous material, bonded by an inorganic binder.

7. A heat resistant means as in Claim 6, wherein said material is formed from inorganic hollow spheroidal powder in the amount 30% to 70% by weight, granular refractory powder in the amount 0 to 30% by weight, refractory fibrous material in the amount 0 to 10% by weight, and sodium silicate solution in the amount 5% to 50% by weight.

8.    A heat resistant means as in Claim 7, wherein the inorganic hollow spheroidal powder has an alumina/silica base.

9.    A heat resistant means as in Claim 7, wherein the granular material is silica sand.

10.    A heat resistant means as in Claim 7, wherein the refractory fibrous material is glass fibre or rock wool.